# EUROPEAN PATENT SPECIFICATION

(11) **EP 4 043 422 B1**
(45) Date of publication and mention of the grant of the patent: **11.09.2024**
(21) Application number: 21880400.3
(22) Date of filing: 07.10.2021
(51) Int. Cl.: C07C 29/141, C07C 31/20, B01J 23/72, B01J 37/02, B01J 21/08

(54) **METHOD FOR PREPARING NEOPENTYL GLYCOL**
VERFAHREN ZUM HERSTELLEN VON NEOPENTYLGLYKOL
PROCÉDÉ DE PRÉPARATION DE NÉOPENTYLGLYCOL

(30) Priority: 16.10.2020 KR 20200134221
(43) Date of publication of application: 17.08.2022
(73) Proprietor: LG Chem, Ltd., Seoul 07336 (KR)
(72) Inventor: YANG, Sungpil, Daejeon 34122 (KR); KIM, Tae Yun, Daejeon 34122 (KR); EOM, Sungshik, Daejeon 34122 (KR); KO, Dong Hyun, Daejeon 34122 (KR); YANG, Jusang, Daejeon 34122 (KR); SHIN, Sangjun, Daejeon 34122 (KR)
(74) Representative: Goddar, Heinz J.
(86) International application number: PCT/KR2021/013765
(87) International publication number: WO 2022/080753

(56) References cited:
- EP-B1- 0 522 368
- WO-A1-2014/120480
- GB-A- 1 219 162
- GB-A- 1 363 677
- KR-A- 20010 033 761
- KR-A- 20130 064 752
- KR-A- 20160 036 486
- KR-A- 20160 133 843
- KR-A- 20170 034 513
- US-A- 4 855 515
- US-A- 5 532 417
- US-A1- 2016 326 073
- US-B2- 8 394 998

## Description

### [Technical Field]

The present application claims priority to and the benefits of Korean Patent Application No.10-2020-0134221, filed with the Korean Intellectual Property Office on October 16, 2020.

The present application relates to a method for producing neopentyl glycol.

### [Background Art]

Neopentyl glycol (NPG) is a white crystalline substance with a melting point of 130 °C or higher, is used as an important intermediate for various synthetic resins, and is being widely used in industries as a raw material for various plastic powder paints, synthetic lubricants, plasticizers, surfactants, textile processing agents, etc.

Such NPG is generally produced by performing aldol condensation reaction of isobutyl aldehyde and formaldehyde to make hydroxypivaldehyde (HPA), and then reacting this HPA with hydrogen under a catalyst.

Conventionally, HPA has been subjected to hydrogenation reaction using a slurry-type Ni-based catalyst. In this case, crude NPG, which is a hydrogenation reaction product, contains 2,2,4-trimethyl-1,3-pentanediol (TMPD), hydroxypivalic acid NPG ester (HPNE), etc. Since TMPD and HPNE have a boiling point very similar to that of NPG, they cannot be separated by simple distillation. Since HPNE is unstable when distilling the reaction mixture and leads to a decrease in the yield of NPG, it is commercially converted to NPG by saponification reaction by adding sodium hydroxide. However, since sodium salts of HPA or other organic acids produced by the saponification reaction promote the decomposition reaction of NPG at a high temperature of 140 °C or higher, the distillation process is constrained. Further, it is impossible to remove TMPD which is not converted to a non-volatile sodium salt when performing the saponification reaction. US 6 545 189 B1 discloses a method for producing neopentyl glycol which comprises hydrogenating HPA in the presence of hydrogen and a hydrogenation catalyst containing nickel or nickel-chromium, in a liquid phase comprising less than 15% water.

Therefore, efforts for producing NPG at a high yield and in an economical way are continuously being made in the art.

### [Disclosure]

### [Technical Problem]

The present application aims to provide a method for producing neopentyl glycol.

### [Technical Solution]

The present application provides a method for producing neopentyl glycol, comprising a step of injecting a hydroxypivaldehyde (HPA) solution and hydrogen into a hydrogenation reactor to perform a hydrogenation reaction, and comprising a step of adjusting the content of H₂O contained in the hydroxypivaldehyde solution to 6.0% by weight or less before the hydroxypivaldehyde solution is injected into the hydrogenation reactor, wherein the hydrogenation reactor is a reactor comprising a catalyst in which copper is supported on a silicon oxide support as a fixed bed.

### [Advantageous Effects]

A method for producing neopentyl glycol according to an embodiment of the present application can slow the aging of the catalyst in the hydrogenation reaction by adjusting the content of H₂O contained in the hydroxypivaldehyde solution injected into the hydrogenation reactor to 6.0% by weight or less.

Accordingly, the method for producing neopentyl glycol according to an embodiment of the present application can increase the period of use of the catalyst in the hydrogenation reaction and reduce the process cost due to catalyst purchase and catalyst replacement.

### [Description of Drawings]

FIG. 1 is a drawing schematically showing a process diagram of a method for producing neopentyl glycol according to an embodiment of the present application.
FIG. 2 is a drawing schematically showing a process diagram of a conventional method for producing neopentyl glycol.

### [Best Mode for Carrying Out the Invention]

Hereinafter, the present specification will be described in more detail.

In the present specification, when a member is said to be located "on" other member, this comprises not only a case in which a member is in contact with other member but also a case in which another member exists between the two members.

In the present specification, when a part "comprises" a certain component, this means that other components may be further comprised rather than excluding other components unless there is a specific opposite description.

As described above, efforts for producing NPG at a high yield and in an economical way are continuously being made in the art.

In particular, during a process for producing neopentyl glycol, H₂O is contained at a high content of about 9 to 12% by weight in a hydroxypivaldehyde (HPA) solution, which is a raw material injected into the reactor. In the process for producing neopentyl glycol, a fixed-bed reactor (FBR) comprising a Cu/SiO₂ catalyst as a fixed bed is used. The present applicant has found out that during the process for producing neopentyl glycol, the FBR operation period can be determined by long-term stability of the Cu/SiO₂ catalyst, and H₂O contained in the hydroxypivaldehyde (HPA) solution may affect the long-term stability of the Cu/SiO₂ catalyst.

Accordingly, during the process of producing neopentyl glycol in the present application, long-term stability of the Cu/SiO₂ catalyst in the FBR has been intended to be secured by adjusting the content of H₂O contained in the hydroxypivaldehyde (HPA) solution injected into the reactor.

A method for producing neopentyl glycol according to the present application comprises a step of performing a hydrogenation reaction by injecting a hydroxypivaldehyde (HPA) solution and hydrogen into a hydrogenation reactor, and comprises a step of adjusting the content of H₂O contained in the hydroxypivaldehyde solution to 6.0% by weight or less before the hydroxypivaldehyde solution is injected into the hydrogenation reactor, wherein the hydrogenation reactor is a reactor comprising a catalyst in which copper is supported on a silicon oxide support as a fixed bed.

In an embodiment of the present application, based on the total weight of the hydroxypivaldehyde solution injected into the hydrogenation reactor, the content of H₂O contained in the hydroxypivaldehyde solution injected into the hydrogenation reactor may be 6.0% by weight or less, 5.5% by weight or less, 5.0% by weight or less, or 0. When the content range of H₂O contained in the hydroxypivaldehyde solution injected into the hydrogenation reactor is satisfied, the aging of the Cu/ SiO₂ catalyst in the hydrogenation reaction can be slowed, and accordingly the period of use of the catalyst in the hydrogenation reaction can be increased, and the process cost due to catalyst purchase and catalyst replacement can be reduced. Further, when the content of H₂O contained in the hydroxypivaldehyde solution injected into the hydrogenation reactor exceeds 6.0% by weight, it is not preferable since long-term stability of the Cu/SiO₂ catalyst in a fixed-bed reactor (FBR) cannot be secured. In particular, considering the content of Si eluted, it is more preferable to adjust the content of H₂O contained in the hydroxypivaldehyde solution injected into the hydrogenation reactor to 5.5% by weight or less.

In an embodiment of the present application, the content of H₂O in the hydroxypivaldehyde solution may be controlled to 6.0% by weight or less using a distillation column.

The distillation column may be a single distillation column or a multi-stage distillation column. For example, the distillation column may be a tray type multi-stage distillation column, but is not limited thereto, and content known in the art may be used. Further, conditions such as temperature, pressure, etc. of the distillation column can be appropriately set by those skilled in the art so as to control the content of H₂O in the hydroxypivaldehyde solution to 6.0% by weight or less as in the embodiment of the present application.

In an embodiment of the present application, the hydroxypivaldehyde solution before the step of adjusting the content of H₂O contained in the hydroxypivaldehyde solution to 6.0% by weight or less may contain 45 to 65% by weight of hydroxypivaldehyde, 1 to 5% by weight of neopentyl glycol, 15 to 35% by weight of alcohol, 8 to 12% by weight of H₂O, and 5 to 15% by weight of a high-boiling point material. Further, the hydroxypivaldehyde solution before the step of adjusting the content of H₂O contained in the hydroxypivaldehyde solution to 6.0% by weight or less may contain 50 to 60% by weight of hydroxypivaldehyde, 1 to 3% by weight of neopentyl glycol, 20 to 30% by weight of alcohol, 8 to 11% by weight of H₂O, and 5 to 10% by weight of a high-boiling point material. The hydroxypivaldehyde solution as described above has an effect of suppressing the formation of byproducts since the heat of reaction can be minimized without deteriorating the reactivity.

The alcohol may be 2-ethylhexanol (2-EH), but is not limited thereto.

The high-boiling point material may comprise one or more of hydroxypivalic hydroxypivalate (HPNE), trimethylpentanediol (TMPD), etc., but is not limited thereto.

In the present application, the hydrogenation reactor is a reactor comprising a catalyst in which copper is supported on a silicon oxide support as a fixed bed. Further, the hydrogenation reactor is be a fixed bed reactor (FBR) filled with the Cu/SiO₂ catalyst, and in this case, there is no need to separate the catalyst and a reaction product, and the reaction temperature and reaction pressure can be lowered than before so that the operation is stable and economical, and the catalyst replacement work is easy and the size of the reactor can be reduced so that there is an effect of greatly reducing the investment cost.

In an embodiment of the present application, at least a portion of a product after the hydrogenation reaction may be recirculated to the hydrogenation reactor. More specifically, a product comprising neopentyl glycol may be recirculated to the hydrogenation reactor, and heat generated in the hydrogenation reaction may be easily controlled accordingly. The hydrogenation reaction may have a recycle ratio (Feed/Recycle) of 0.1 to 8.0 kg/kg, 1 to 7 kg/kg, or 2 to 6 kg/kg, and there are effects of easily controlling the reaction heat and preventing a runaway reaction within the range. The "recycle ratio (Feed/Recycle)" refers to raw material supply flow amount to recirculation flow amount, unless otherwise specified.

In an embodiment of the present application, in the step of performing a hydrogenation reaction by injecting a hydroxypivaldehyde (HPA) solution and hydrogen into the hydrogenation reactor, the hydrogenation reaction may have a reaction temperature of 100 to 250 °C, 100 to 200 °C, or 100 to 180 °C.

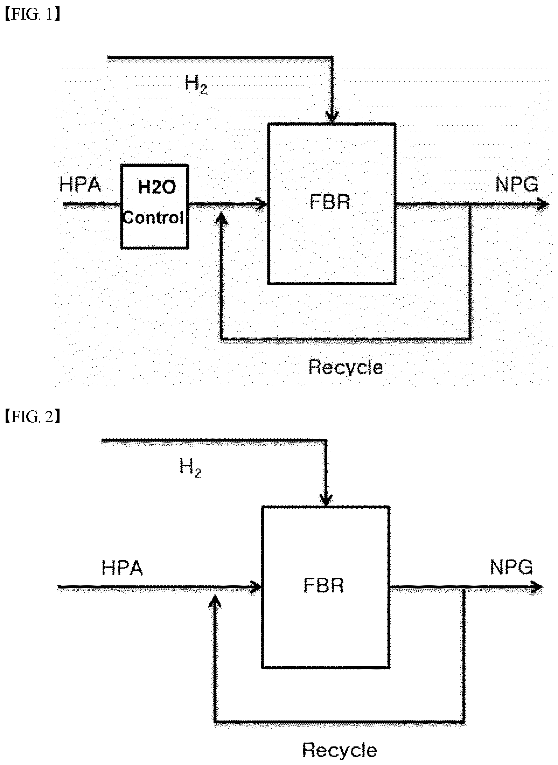

A process diagram of a method for producing neopentyl glycol according to an embodiment of the present application is schematically shown in FIG. 1 below, and a process diagram of a conventional method for producing neopentyl glycol is schematically shown in FIG. 2 below. As described in FIGS. 1 and 2 below, the method for producing neopentyl glycol according to the present application comprises a step of performing a hydrogenation reaction by injecting a hydroxypivaldehyde (HPA) solution and hydrogen into a hydrogenation reactor, wherein the hydrogenation reactor is a reactor comprising a catalyst in which copper is supported on a silicon oxide support as a fixed bed, and comprises a step of adjusting the content of H₂O contained in the hydroxypivaldehyde solution to 6.0% by weight or less before the hydroxypivaldehyde solution is injected into the hydrogenation reactor.

In the method for producing neopentyl glycol according to the present application, the aging of the Cu/SiO₂ catalyst in the hydrogenation reaction can be slowed by adjusting the content of H₂O contained in the hydroxypivaldehyde solution injected into the hydrogenation reactor to 6.0% by weight or less.

Accordingly, the method for producing neopentyl glycol according to an embodiment of the present application can increase the period of use of the Cu/SiO₂ catalyst in the hydrogenation reaction, and can reduce the process cost due to catalyst purchase and catalyst replacement.

### [Mode for Carrying Out the Invention]

Hereinafter, Examples will be given and described in detail in order to describe the present application specifically. However, Examples according to the present application may be modified in various other forms, and the scope of the present application is not to be construed as being limited to Examples described in detail below. Examples of the present application are provided to more completely explain the present application to those of ordinary skill in the art.

### <Example>

### <Example 1>

An HPA solution (55% by weight of HPA, 2% by weight of NPG, 25% by weight of 2-ethylhexanol, 8.7% by weight of H₂O, 9.3% by weight of a high-boiling point material) was prepared.

After controlling the content of H₂O in the HPA solution to 5.49% by weight, it was injected into a fixed bed reactor (FBR) filled with a Cu/SiO₂ catalyst, and a hydrogenation reaction was carried out at 30 bar and 110 °C to produce neopentyl glycol. The content of H₂O in the HPA solution was controlled using a distillation column.

### <Example 2>

The production process was carried out in the same manner as in Example 1 except that the content of H₂O in the HPA solution injected into the FBR was controlled to 5.02% by weight.

### <Example 3>

The production process was carried out in the same manner as in Example 1 except that the content of H₂O in the HPA solution injected into the FBR was controlled to 6.0% by weight.

### <Comparative Example 1>

The production process was carried out in the same manner as in Example 1 except that the process of controlling the content of H₂O in the HPA solution injected into the FBR was excluded. At this time, the content of H₂O in the HPA solution injected into the FBR was 8.7% by weight.

### <Experimental Example>

The reaction products produced in Examples and Comparative Example were analyzed by Inductively Coupled Plasma-Optical Emission Spectroscopy (ICP-OES) to measure Si concentrations, and the measurement results are shown in Table 1 below.

The ICP-OES analysis method is as follows.
1) About 10 g of samples was accurately measured in a platinum crucible.
2) The samples were heated on a hot plate to concentrate solvent components.
3) 1 mL of nitric acid was put into the samples and heated.
4) The samples were heated and dried.
5) 1mL of concentrated nitric acid was added to the samples, and 200 µl of hydrogen peroxide was added to the samples (the process was performed 2 to 3 times).
6) When the organic materials were dissolved, 1 mL of nitric acid was put into the samples and heated.
7) When the samples were clearly dissolved, they were diluted with 10 mL of ultrapure water.
8) The samples were analyzed with ICP-OES (PERKIN-ELMER, OPTIMA 8300DV).

**[Table 1]**

| | Si content (ppm) |
|---|---|
| Example 1 | 2.3 |
| Example 2 | 1.3 |
| Example 3 | 3.0 |
| Comparative Example 1 | 5.4 |

As the results of Table 1 above, it can be confirmed that there is a large amount of Si elution from the Cu/SiO₂ catalyst in the process for producing neopentyl glycol as in Comparative Example 1 so that it may cause a problem in long-term stability of the catalyst.

As shown in the above results, the method for producing neopentyl glycol according to an embodiment of the present application can slow the aging of the catalyst in the hydrogenation reaction by adjusting the content of H₂O contained in the hydroxypivaldehyde solution injected into the hydrogenation reactor, wherein the hydrogenation reactor is a reactor comprising a catalyst in which copper is supported on a silicon oxide support as a fixed bed, to 6.0% by weight or less. In particular, considering the content of Si eluted, it is more preferable to adjust the content of H₂O contained in the hydroxypivaldehyde solution injected into the hydrogenation reactor to 5.5% by weight or less.

Accordingly, the method for producing neopentyl glycol according to an embodiment of the present application can increase the period of use of the Cu/SiO₂ catalyst in the hydrogenation reaction, and can reduce the process cost due to catalyst purchase and catalyst replacement.

## Claims

1. A method for producing neopentyl glycol, the method comprising a step of performing a hydrogenation reaction by injecting a hydroxypivaldehyde (HPA) solution and hydrogen into a hydrogenation reactor, and comprising a step of adjusting the content of H₂O contained in the hydroxypivaldehyde solution to 6.0% by weight or less before the hydroxypivaldehyde solution is injected into the hydrogenation reactor,
wherein the hydrogenation reactor is a reactor comprising a catalyst in which copper is supported on a silicon oxide support as a fixed bed.

2. The method of claim 1, wherein the step of adjusting the content of H₂O contained in the hydroxypivaldehyde solution to 6.0% by weight or less uses a distillation column.

3. The method of claim 1, wherein the hydroxypivaldehyde solution before the step of adjusting the content of H₂O contained in the hydroxypivaldehyde solution to 6.0% by weight or less contains 45 to 65% by weight of hydroxypivaldehyde, 1 to 5% by weight of neopentyl glycol, 15 to 35% by weight of alcohol, 8 to 12% by weight of H₂O, and 5 to 15% by weight of a high-boiling point material.

4. The method of claim 1, wherein at least a portion of a product after the hydrogenation reaction is recirculated to the hydrogenation reactor.

5. The method of claim 1, wherein the hydrogenation reaction has a reaction temperature of 100 to 250 °C.

## Patentansprüche

1. Verfahren zur Herstellung von Neopentylglycol, wobei das Verfahren einen Schritt des Durchführens einer Hydrierungsreaktion durch Injizieren einer Hydroxypivalaldehyd(HPA)-Lösung und von Wasserstoff in einen Hydrierungsreaktor umfasst und einen Schritt des Einstellens des Gehalts an H₂O, das in der Hydroxypivalaldehyd-Lösung enthalten ist, auf 6,0 Gew.-% oder weniger, bevor die Hydroxypivalaldehyd-Lösung in den Hydrierungsreaktor injiziert wird, umfasst,
wobei der Hydrierungsreaktor ein Reaktor ist, der einen Katalysator umfasst, in dem Kupfer auf einem Siliciumoxidträger als ein Festbett geträgert ist.

2. Verfahren nach Anspruch 1, bei dem der Schritt des Einstellens des Gehalts an H₂O, das in der Hydroxypivalaldehyd-Lösung enthalten ist, auf 6,0 Gew.-% oder weniger eine Destillationskolonne verwendet.

3. Verfahren nach Anspruch 1, bei dem die Hydroxypivalaldehyd-Lösung vor dem Schritt des Einstellens des Gehalts an H₂O, das in der Hydroxypivalaldehyd-Lösung enthalten ist, auf 6,0 Gew.-% oder weniger 45 bis 65 Gew.-% Hydroxypivalaldehyd, 1 bis 5 Gew.-% Neopentylglycol, 15 bis 35 Gew.-% Alkohol, 8 bis 12 Gew.-% H₂O und 5 bis 15 Gew.-% eines Materials mit hohem Siedepunkt enthält.

4. Verfahren nach Anspruch 1, bei dem wenigstens ein Teil eines Produkts nach der Hydrierungsreaktion in den Hydrierungsreaktor zurückgeführt wird.

5. Verfahren nach Anspruch 1, bei dem die Hydrierungsreaktion eine Reaktionstemperatur von 100 bis 250 °C aufweist.

## Revendications

1. Procédé de production de néopentylglycol, le procédé comprenant une étape consistant à effectuer une réaction d'hydrogénation en injectant une solution d'hydroxypivaldéhyde (HPA) et de l'hydrogène dans un réacteur d'hydrogénation, et comprenant une étape consistant à ajuster la teneur en H₂O contenue dans la solution d'hydroxypivaldéhyde à 6,0 % en poids ou moins avant que la solution d'hydroxypivaldéhyde ne soit injectée dans le réacteur d'hydrogénation,
dans lequel le réacteur d'hydrogénation est un réacteur comprenant un catalyseur dans lequel le cuivre est supporté sur un support d'oxyde de silicium en tant que lit fixe.

2. Procédé selon la revendication 1, dans lequel l'étape d'ajustement de la teneur en H₂O contenue dans la solution d'hydroxypivaldéhyde à 6,0 % en poids ou moins utilise une colonne de distillation.

3. Procédé selon la revendication 1, dans lequel la solution d'hydroxypivaldéhyde avant l'étape d'ajustement de la teneur en H₂O contenue dans la solution d'hydroxypivaldéhyde à 6,0 % en poids ou moins contient 45 à 65 % en poids d'hydroxypivaldéhyde, 1 à 5 % en poids de néopentylglycol, 15 à 35 % en poids d'alcool, 8 à 12 % en poids de H₂O et 5 à 15 % en poids d'une matière à point d'ébullition élevé.

4. Procédé selon la revendication 1, dans lequel au moins une partie d'un produit après la réaction d'hydrogénation est recirculée dans le réacteur d'hydrogénation.

5. Procédé selon la revendication 1, dans lequel la réaction d'hydrogénation a une température de réaction comprise entre 100 et 250 °C.
